(19) **Europäisches Patentamt**
**European Patent Office**
**Office européen des brevets**

(11) **EP 1 539 265 B1**

(12) **EUROPEAN PATENT SPECIFICATION**

(45) Date of publication and mention
of the grant of the patent:
**26.09.2007 Bulletin 2007/39**

(51) Int Cl.:
**A61L 27/54** *(2006.01)*      **A61L 27/26** *(2006.01)*
**A61L 31/16** *(2006.01)*      **A61L 31/04** *(2006.01)*

(21) Application number: **03785264.7**

(22) Date of filing: **13.08.2003**

(86) International application number:
**PCT/US2003/025362**

(87) International publication number:
**WO 2004/014450 (19.02.2004 Gazette 2004/08)**

(54) **ACTIVE AGENT DELIVERY SYSTEM INCLUDING A HYDROPHOBIC CELLULOSE DERIVATE**

SYSTEM ZUR ABGABE VON WIRKSTOFFEN MIT EINEM HYDROPHOBEN CELLULOSE-DERIVAT

SYSTEME D'ADMINISTRATION DE PRINCIPE ACTIF COMPRENANT UN DERIVE CELLULOSIQUE HYDROPHOBE

(84) Designated Contracting States:
**AT BE BG CH CY CZ DE DK EE ES FI FR GB GR HU IE IT LI LU MC NL PT RO SE SI SK TR**

(30) Priority: **13.08.2002 US 403477 P**

(43) Date of publication of application:
**15.06.2005 Bulletin 2005/24**

(73) Proprietor: **MEDTRONIC, INC.**
**Minneapolis, MN 55432 (US)**

(72) Inventors:
• **SPARER, Randall, V.**
**Andover, MN 55304 (US)**
• **HOBOT, Christopher, M.**
**Tonka Bay, MN 55331 (US)**

• **LYU, SuPing**
**Maple Grove, MN 55311 (US)**
• **DANG, Kiem**
**Blaine, MN 55449 (US)**
• **CHENG, Peiwen**
**Santa Rosa, CA 95409 (US)**

(74) Representative: **Thomson, James B.**
**Frank B. Dehn & Co.**
**St Bride's House**
**10 Salisbury Square**
**London EC4Y 8JD (GB)**

(56) References cited:
EP-A- 0 281 482      US-A- 5 676 972
US-A- 6 077 916

EP 1 539 265 B1

**Description**

BACKGROUND OF THE INVENTION

A polymeric coating on a medical device may serve as a repository for delivery of an active agent (e.g., a therapeutic agent) to a subject. For many such applications, polymeric coatings must be as thin as possible. Polymeric materials for use in delivering an active agent may also be in various three-dimensional shapes.

Conventional active agent delivery systems suffer from limitations that include structural failure due to cracking and delamination from the device surface. Furthermore, they tend to be limited in terms of the range of active agents that can be used, the range of amounts of active agents that can be included within a delivery system, and the range of the rates at which the included active agents are delivered therefrom. This is frequently because many conventional systems include a single polymer.

Thus, there is a continuing need for active agent delivery systems with greater versatility and tunability.

SUMMARY OF THE INVENTION

The present invention provides active agent delivery systems that have generally good versatility and tunability in controlling the delivery of active agents. Typically, such advantages result from the use of a blend of two or more miscible polymers. These delivery systems can be incorporated into medical devices, e.g., stents, stent grafts, anastomotic connectors, if desired.

The active agent delivery systems of the present invention typically include a blend of at least two miscible polymers, wherein at least one polymer (preferably one of the miscible polymers) is matched to the solubility of the active agent such that the delivery of the active agent preferably occurs predominantly under permeation control. In this context, "predominantly" with respect to permeation control means that at least 50%, preferably at least 75%, and more preferably at least 90%, of the total active agent load is delivered by permeation control.

Permeation control is typically important in delivering an active agent from systems in which the active agent passes through a miscible polymer blend having a "critical" dimension on a micron-scale level (i.e., the net diffusion path is no greater than about 1000 micrometers, although for shaped objects it can be up to about 10,000 microns). Furthermore, it is generally desirable to select polymers for a particular active agent that provide desirable mechanical properties without being detrimentally affected by nonuniform incorporation of the active agent.

In one preferred embodiment, the present invention provides an active agent delivery system that includes an active agent and a miscible polymer blend that includes a hydrophobic cellulose derivative and a polyvinyl homopolymer or copolymer selected from the group consisting of a polyvinyl alkylate homopolymer or copolymer, a polyvinyl alkyl ether homopolymer or copolymer, a polyvinyl acetal homopolymer or copolymer, and combinations thereof.

In another preferred embodiment, the present invention provides an active agent delivery system that includes an active agent and a miscible polymer blend that includes a hydrophobic cellulose derivative and a polyvinyl homopolymer or copolymer, wherein: the polyvinyl homopolymer or copolymer is selected from the group consisting of a polyvinyl alkylate homopolymer or copolymer, a polyvinyl alkyl ether homopolymer or copolymer, a polyvinyl acetal homopolymer or copolymer, and combinations thereof; the active agent that is hydrophobic and has a molecular weight of no greater than (i.e., less than or equal to) about 1200 grams per mole (g/mol); each of the active agent, the hydrophobic cellulose derivative, and the polyvinyl homopolymer or copolymer has a solubility parameter; the difference between the solubility parameter of the active agent and the solubility parameter of the hydrophobic cellulose derivative is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$), and the difference between the solubility parameter of the active agent and at least one solubility parameter of the polyvinyl homopolymer or copolymer thereof is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$); and the difference between the solubility parameter of the hydrophobic cellulose derivative and at least one solubility parameter of the polyvinyl homopolymer or copolymer thereof is no greater than about 5 $J^{1/2}/cm^{3/2}$ (preferably, no greater than about 3 $J^{1/2}/cm^{3/2}$).

The present invention also provides medical devices that include such active agent delivery systems.

In one preferred embodiment, a medical device is provided that includes: a substrate surface; a polymeric undercoat layer adhered to the substrate surface; and a polymeric top coat layer adhered to the polymeric undercoat layer; wherein the polymeric top coat layer includes an active agent incorporated within a miscible polymer blend that includes a hydrophobic cellulose derivative and a polyvinyl homopolymer or copolymer selected from the group consisting of a polyvinyl alkylate homopolymer or copolymer, a polyvinyl alkyl ether homopolymer or copolymer, a polyvinyl acetal homopolymer or copolymer, and combinations thereof.

In another preferred embodiment, a stent is provided that includes: a substrate surface; a polymeric undercoat layer adhered to the substrate surface; and a polymeric top coat layer adhered to the undercoat layer; wherein the polymeric top coat layer includes an active agent incorporated within a miscible polymer blend that includes a hydrophobic cellulose derivative and a polyvinyl homopolymer or copolymer selected from the group consisting of a polyvinyl alkylate homopolymer or copolymer, a polyvinyl alkyl ether homopolymer or copolymer, a polyvinyl acetal homopolymer or copolymer, and combinations thereof.

The present invention also provides methods for making an active agent delivery system.

In one embodiment, a method of forming an active agent delivery system includes: combining a hydrophobic cellulose derivative and a polyvinyl homopolymer or copolymer to form a miscible polymer blend, wherein the polyvinyl homopolymer or copolymer is selected from the group consisting of a polyvinyl alkylate homopolymer or copolymer, a polyvinyl alkyl ether homopolymer or copolymer, a polyvinyl acetal homopolymer or copolymer, and combinations thereof; and combining an active agent with the miscible polymer blend.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

BRIEF DESCRIPTION OF THE DRAWINGS

Figure 1A-D. TSC scans of polyvinyl acetate and cellulose acetate butyrate blends (PVAC/CAB). The transition peaks shifted depending on the blend composition.

Figure 2. DSC scans of PVAC/CAB blends. The glass transitions of the blends changed as a function of the PVAC content of the blends.

Figure 3. Graph of cumulative release of dexamethasone from various PVAC/CAB blends versus the square root of time. The release rates were tuned by changing the amount of PVAC in the blends.

Figure 4. Graph of diffusion coefficient of dexamethasone in PVAC/CAB blends versus the composition of the blend. The diffusion coefficient increased as a function of the PVAC content of the blends.

DETAILED DESCRIPTION OF ILLUSTRATIVE EMBODIMENTS

The present invention provides active agent delivery systems that include an active agent for delivery to a subject and a miscible polymer blend. The delivery systems can include a variety of polymers as long as at least two are miscible as defined herein. The active agent may be incorporated within the miscible polymer blend such that it is dissoluted from the blend, or the blend can initially function as a barrier to the environment through which the active agent passes.

Miscible polymer blends are advantageous because they can provide greater versatility and tunability for a greater range of active agents than can conventional systems that include immiscible mixtures or only a single polymer, for example. That is, using two or more polymers, at least two of which are miscible, can generally provide a more versatile active agent delivery system than a delivery system with only one of the polymers. A greater range of types of active agents can typically be used. A greater range of amounts of an active agent can typically be incorporated into and delivered from (preferably, predominantly under permeation control) the delivery systems of the present invention. A greater range of delivery rates for an active agent can typically be provided by the delivery systems of the present invention. At least in part, this is because of the use of a miscible polymer blend that includes at least two miscible polymers. It should be understood that, although the description herein refers to two polymers, the invention encompasses systems that include more than two polymers, as long as a miscible polymer blend is formed that includes at least two miscible polymers.

A miscible polymer blend of the present invention has a sufficient amount of at least two miscible polymers to form a continuous portion, which helps tune the rate of release of the active agent. Such a continuous portion (i.e., continuous phase) can be identified microscopically or by selective solvent etching. Preferably, the at least two miscible polymers form at least 50 percent by volume of a miscible polymer blend.

A miscible polymer blend can also optionally include a dispersed (i.e., discontinuous) immiscible portion. If both continuous and dispersed portions are present, the active agent can be incorporated within either portion. Preferably, the active agent is loaded into the continuous portion to provide delivery of the active agent predominantly under permeation control. To load the active agent, the solubility parameters of the active agent and the portion of the miscible polymer blend a majority of the active agent is loaded into are matched (typically to within no greater than about 10 $J^{1/2}/cm^{3/2}$, preferably, no greater than about 5 $J^{1/2}/cm^{3/2}$, and more preferably, no greater than about 3 $J^{1/2}/cm^{3/2}$). The continuous phase controls the release of the active agent regardless of where the active agent is loaded.

A miscible polymer blend, as used herein, encompasses a number of completely miscible blends of two or more polymers as well as partially miscible blends of two or more polymers. A completely miscible polymer blend will ideally have a single glass transition temperature (Tg) due to mixing at the molecular level over the entire concentration range. Partially miscible polymer blends may have multiple Tg's because mixing at the molecular level is limited to only parts of the entire concentration range. These partially miscible blends are included within the scope of the term "miscible polymer blend" as long as the absolute value of the difference between at least one Tg ($Tg_{Polymer\ 1}$-$Tg_{polymer\ 2}$) for each of at least two polymers within the blend is reduced by the act of blending. Tg's can be determined by measuring the mechanical properties, thermal properties, electric properties, etc. as a function of temperature.

A miscible polymer blend can also be determined based on its optical properties. A completely miscible blend forms a stable and homogeneous domain that is transparent, whereas an immiscible blend forms a heterogeneous domain that scatters light and visually appears turbid unless the components have identical refractive indices. Additionally, a phase-separated structure of immiscible blends can be directly observed with microscopy. A simple method used in the present invention to check the miscibility involves mixing the polymers and forming a thin film of about 10 micrometers to about 50 micrometers thick. If such a film is generally as clear and transparent as the least clear and transparent film of the same thickness of the individual polymers prior to blending, then the polymers are completely miscible.

Miscibility between polymers depends on the interactions between them and their molecular structures and molecular weights. The interaction between polymers can be characterized by the so-called Flory-Huggins parameter ($\chi$). When $\chi$ is close to zero (0) or even is negative, the polymers are very likely miscible. Theoretically, $\chi$ can be estimated from the solubility parameters of the polymers, i.e. $\chi$, is proportional to the squared difference between them. Therefore, the miscibility of polymers can be approximately predicted. For example, the closer the solubility parameters of the two polymers are the higher the possibility that the two polymers are miscible. Miscibility between polymers tends to decrease as their molecular weights increases.

Thus, in addition to the experimental determinations, the miscibility between polymers can be predicted simply based on the Flory-Huggins interaction parameters, or even more simply, based the solubility parameters of the components. However, because of the molecular weight effect, close solubility parameters do not necessarily guarantee miscibility.

It should be understood that a mixture of polymers needs only to meet one of the definitions provided herein to be miscible. Furthermore, a mixture of polymers may become a miscible blend upon incorporation of an active agent. The types and amounts of polymers and active agents are typically selected to form a system having a preselected dissolution time (or rate) through a preselected critical dimension of the miscible polymer blend. Glass transition temperatures and solubility parameters can be used in guiding one of skill in the art to select an appropriate combination of components in an active agent delivery system, whether the active agent is incorporated into the miscible polymer blend or not. Solubility parameters are generally useful for determining miscibility of the polymers and matching the solubility of the active agent to that of the miscible polymer blend. Glass transition temperatures are generally useful for determining miscibility of the polymers and tuning the dissolution time (or rate) of the active agent. These concepts are discussed in greater detail below.

A miscible polymer blend can be used in combination with an active agent in the delivery systems of the present invention in a variety of formats as long as the miscible polymer blend controls the delivery of the active agent.

In one embodiment, a miscible polymer blend has an active agent incorporated therein. Preferably, such an active agent is dissolved predominantly under permeation control, which requires at least some solubility of the active agent in the continuous portion (i.e., the miscible portion) of the polymer blend, whether the majority of the active agent is loaded in the continuous portion or not. Dispersions are acceptable as long as little or no porosity channeling occurs during dissolution of the active agent and the size of the dispersed domains is much smaller than the critical dimension of the blends, and the physical properties are generally uniform throughout the composition for desirable mechanical performance. This embodiment is often referred to as a "matrix" system.

In another embodiment, a miscible polymer blend initially provides a barrier to permeation of an active agent. This embodiment is often referred to as a "reservoir" system. A reservoir system can be in many formats with two or more layers. For example, a miscible polymer blend can form an outer layer over an inner layer of another material (referred to herein as the inner matrix material). In another example, a reservoir system can be in the form of a core-shell, wherein the miscible polymer blend forms the shell around the core matrix (i.e., the inner matrix material). At least initially upon formation, the miscible polymer blend in the shell or outer layer could be substantially free of active agent. Subsequently, the active agent permeates from the inner matrix and through the miscible polymer blend for delivery to the subject. The inner matrix material can include a wide variety of conventional materials used in the delivery of active agents. These include, for example, an organic polymer such as those described herein for use in the miscible polymer blends, or a wax, or a different miscible polymer blend. Alternatively, the inner matrix material can be the active agent itself.

For a reservoir system, the release rate of the active agent can be tuned with selection of the material of the outer layer. The inner matrix can include an immiscible mixture of polymers or it can be a homopolymer if the outer layer is a miscible blend of polymers.

As with matrix systems, an active agent in a reservoir system is preferably dissolved predominantly under

permeation control through the miscible polymer blend of the barrier layer (i.e., the barrier polymer blend), which requires at least some solubility of the active agent in the barrier polymer blend. Again, dispersions are acceptable as long as little or no porosity channeling occurs in the barrier polymer blend during dissolution of the active agent and the size of the dispersed domains is much smaller than the critical dimension of the blends, and the physical properties are generally uniform throughout the barrier polymer blend for desirable mechanical performance. Although these considerations may also be desirable for the inner matrix, they are not necessary requirements.

Typically, the amount of active agent within an active agent delivery system of the present invention is determined by the amount to be delivered and the time period over which it is to be delivered. Other factors can also contribute to the level of active agent present, including, for example, the ability of the composition to form a uniform film on a substrate.

Preferably, for a matrix system, an active agent is present within (i.e., incorporated within) a miscible polymer blend in an amount of at least about 0.1 weight percent (wt-%), more preferably, at least about 1 wt-%, and even more preferably, at least about 5 wt-%, based on the total weight of the miscible polymer blend and the active agent. Preferably, for a matrix system, an active agent is present within a miscible polymer blend in an amount of no greater than about 80 wt-%, more preferably, no greater than about 50 wt-%, and most preferably, no greater than about 30 wt-%, based on the total weight of the miscible polymer blend and the active agent. Typically and preferably, the amount of active agent will be at or below its solubility limit in the miscible polymer blend.

Preferably, for a reservoir system, an active agent is present within an inner matrix in an amount of at least about 0.1 wt-%, more preferably, at least about 10 wt-%, and even more preferably, at least about 25 wt-%, based on the total weight of the inner matrix (including the active agent). Preferably, for a reservoir system, an active agent is present within an inner matrix in an amount of up to 100 wt-%, and more preferably, no greater than about 80 wt-%, based on the total weight of the inner matrix (including the active agent).

In the active agent delivery systems of the present invention, an active agent is dissolutable through a miscible polymer blend. Dissolution is preferably controlled predominantly by permeation of the active agent through the miscible polymer blend. That is, the active agent initially dissolves into the miscible polymer blend and then diffuses through the miscible polymer blend predominantly under permeation control. Thus, as stated above, for certain preferred embodiments, the active agent is at or below the solubility limit of the miscible polymer blend. Although not wishing to be bound by theory, it is believed that because of this mechanism the active agent delivery systems of the present invention have a significant level of tunability.

If the active agent exceeds the solubility of the miscible polymer blend and the amount of insoluble active agent exceeds the percolation limit, then the active agent could be dissoluted predominantly through a porosity mechanism. In addition, if the largest dimension of the active agent insoluble phase (e.g., particles or aggregates of particles) is on the same order as the critical dimension of the miscible polymer blend, then the active agent could be dissoluted predominantly through a porosity mechanism. Dissolution by porosity control is typically undesirable because it does not provide effective predictability and controllability.

Because the active agent delivery systems of the present invention preferably have a critical dimension on the micron-scale level, it can be difficult to include a sufficient amount of active agent and avoid delivery by a porosity mechanism. Thus, the solubility parameters of the active agent and at least one polymer of the miscible polymer blend are matched to maximize the level of loading while decreasing the tendency for delivery by a porosity mechanism.

One can determine if there is a permeation-controlled release mechanism by examining a dissolution profile of the amount of active agent released versus time (t). For permeation-controlled release from a matrix system, the profile is directly proportional to $t^{1/2}$. For permeation-controlled release from a reservoir system, the profile is directly proportional to t. Alternatively, under sink conditions (i.e., conditions under which there are no rate-limiting barriers between the polymer blend and the media into which the active agent is dissoluted), porosity-controlled dissolution could result in a burst effect (i.e., an initial very rapid release of active agent).

The active agent delivery systems of the present invention, whether in the form of a matrix system or a reservoir system, for example, without limitation, can be in the form of coatings on substrates (e.g., open or closed cell foams, woven or nonwoven materials), films (which can be free-standing as in a patch, for example), shaped objects (e.g., microspheres, beads, rods, fibers, or other shaped objects), wound packing materials, etc.

As used herein, an "active agent" is one that produces a local or systemic effect in a subject (e.g., an animal). Typically, it is a pharmacologically active substance. The term is used to encompass any substance intended for use in the diagnosis, cure, mitigation, treatment, or prevention of disease or in the enhancement of desirable physical or mental development and conditions in a subject. The term "subject" used herein is taken to include humans, sheep, horses, cattle, pigs, dogs, cats, rats, mice, birds, reptiles, fish, insects, arachnids, protists (e.g., protozoa), and prokaryotic bacteria. Preferably, the subject is a human or other mammal.

Active agents can be synthetic or naturally occurring and include, without limitation, organic and inorganic chemical agents, polypeptides (which is used herein to encompass a polymer of L- or D- amino acids of any length including peptides, oligopeptides, proteins, enzymes, hormones, etc.), polynucleotides (which is used herein to encompass a polymer of nucleic acids of any length including oligonucleotides, single-and double-stranded DNA, single- and double-stranded RNA, DNA/RNA chimeras, etc.), saccharides (e.g., mono-, di-, poly-saccharides, and mucopolysaccharides), vitamins, viral agents, and other living material, radionuclides, and the like. Examples include antithrombogenic and anticoagulant agents such as heparin, coumadin, coumarin, protamine, and hirudin; antimicrobial agents such as antibiotics; antineoplastic agents and anti-proliferative agents such as etoposide, podophylotoxin; antiplatelet agents including aspirin and dipyridamole; antimitotics (cytotoxic agents) and antimetabolites such as methotrexate, colchicine, azathioprine, vincristine, vinblastine, fluorouracil, adriamycin, and mutamycinnucleic acids; antidiabetic such as rosiglitazone maleate; and anti-inflammatory agents. Anti-inflammatory agents for use in the present invention include glucocorticoids, their salts, and derivatives thereof, such as cortisol, cortisone, fludrocortisone, Prednisone, Prednisolone, 6α-methylprednisolone, triamcinolone, betamethasone, dexamethasone, beclomethasone, aclomethasone, amcinonide, clebethasol, and clocortolone. Preferably, the active agent is not heparin.

For preferred active agent delivery systems of the present invention, the active agent is typically matched to the solubility of the miscible portion of the polymer blend. For the present invention, at least one polymer of the polymer blend is hydrophobic. Thus, preferred active agents for the present invention are hydrophobic. Preferably, if the active agent is hydrophobic, then at least one of the miscible polymers is hydrophobic, and if the active agent is hydrophilic, then at least one of the miscible polymers is hydrophilic, although this is not necessarily required.

As used herein, in this context (in the context of the polymer of the blend), the term "hydrophobic" refers to a material that will not increase in volume by more than 10% or in weight by more than 10%, whichever comes first, when swollen by water at body temperature (i.e., about 37°C). In contrast, the term "hydrophilic" refers to a material that will increase in volume by at least 10% or in weight by at least 10%, whichever comes first, when swollen by water at body temperature (i.e., about 37°C).

As used herein, in this context (in the context of the active agent), the term "hydrophobic" refers to an active agent that has a solubility in water at room temperature (i.e., about 25°C) of no more than (i.e., less than or equal to) 200 micrograms per milliliter. In contrast, the term "hydrophilic" refers to an active agent that has a solubility in water of more than 200 micrograms per milliliter.

For delivery systems in which the active agent is hydrophobic, regardless of the molecular weight, polymers are typically selected such that the molar average solubility parameter of the miscible polymer blend is no greater than 28 $J^{1/2}/cm^{3/2}$ (preferably, no greater than 25 $J^{1/2}/cm^{3/2}$). For delivery systems in which the active agent is hydrophilic, regardless of the molecular weight, polymers are typically selected such that the molar average solubility parameter of the miscible polymer blend is greater than 21 $J^{1/2}/cm^{3/2}$ (preferably, greater than 25 $J^{1/2}/cm^{3/2}$). Herein "molar average solubility parameter" means the average of the solubility parameters of the blend components that are miscible with each other and that form the continuous portion of the miscible polymer blend. These are weighted by their molar percentage in the blend, without the active agent incorporated into the polymer blend.

As the size of the active agent gets sufficiently large, diffusion through the polymer is affected. Thus, active agents can be categorized based on molecular weights and polymers can be selected depending on the range of molecular weights of the active agents.

For preferred active agent delivery systems of the present invention, the active agent has a molecular weight of no greater than about 1200 g/mol. For even more preferred embodiments, active agents of a molecular weight no greater than about 800 g/mol are desired.

Of the active agents listed above, those that are hydrophobic and have a molecular weight of no greater than about 1200 g/mol are particularly preferred.

As stated above, the types and amounts of polymers and active agents are typically selected to form a system having a preselected dissolution time (t) through a preselected critical dimension (x) of the miscible polymer blend. This involves selecting at least two polymers to provide a target diffusivity, which is directly proportional to the critical dimension squared divided by the time ($x^2/t$), for a given active agent.

The diffusivity can be easily measured by dissolution analysis using the following equation (see, for example, Kinam Park edited, Controlled Drug Delivery: Challenges and Strategies, American Chemical Society, Washington, DC, 1997):

$$D = (\frac{M_t}{4M_\infty})^2 \cdot \frac{\pi x^2}{t}$$

wherein D = diffusion coefficient; $M_t$ = cumulative release; $M_\infty$ = total loading of active agent; x = the critical dimension (e.g., thickness of the film); and t = the dissolution time. This equation is valid during dissolution of up to 60 percent by weight of the initial load of the active agent. Also, blend samples should be in the form of a film.

In refining the selection of the polymers for the desired active agent, the desired dissolution time (or rate), and the desired critical dimension, the parameters that can be considered when selecting the polymers for the desired active agent include glass transition temperatures of the polymers, solubility parameters of the polymers, and solubility parameters of the active agents. These can be used in guiding one of skill in the art to select an appropriate combination of components in an active agent delivery system, whether the active agent is incorporated into the miscible polymer blend or not.

For enhancing the tunability of a permeation-controlled delivery system, for example, preferably the polymers are selected such that the difference between at least one Tg of at least two of the polymers of the blend is sufficient to provide the target diffusivity. The target diffusivity is determined by the preselected dissolution time (t) for delivery and the preselected critical dimension (x) of the polymer composition and is directly proportional to $x^2/t$.

For enhancing the versatility of a permeation-controlled delivery system, for example, preferably the polymers are selected such that at least one of the following relationships is true: (1) the difference between the solubility parameter of the active agent and at least one solubility parameter of at least one polymer is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$) ; and (2) the difference between at least one solubility parameter of each at least two polymers is no greater than about 5 $J^{1/2}/cm^{3/2}$ (preferably, no greater than about 3 $J^{1/2}/cm^{3/2}$). More preferably, both relationships are true. Most preferably, both relationships are true for all polymers of the blend.

Typically, a compound has only one solubility parameter, although certain polymers, such as segmented copolymers and block copolymers, for example, can have more than one solubility parameter. Solubility parameters can be measured or they are calculated using an average of the values calculated using the Hoy Method and the Hoftyzer-van Krevelen Method (chemical group contribution methods), as disclosed in D.W. van Krevelen, Properties of Polymers, 3rd Edition, Elsevier, Amsterdam. To calculate these values, the volume of each chemical is needed, which can be calculated using the Fedors Method, disclosed in the same reference.

Solubility parameters can also be calculated with computer simulations, for example, molecular dynamics simulation and Monte Carlo simulation. Specifically, the molecular dynamics simulation can be conducted with Accelrys Materials Studio, Accelrys Inc., San Diego, CA. The computer simulations can be used to directly calculate the Flory-Huggins parameter.

A miscible polymer blend of the present invention includes a hydrophobic cellulose derivative. A hydrophobic cellulose derivative is preferably present in the miscible polymer blend in an amount of at least about 0.1 wt-%, and more preferably up to about 99.9 wt-%, based on the total weight of the blend, depending on the active agent and specific choice of polymers.

Preferred examples of a hydrophobic cellulose derivative include esters (organic or inorganic) and ethers. Preferred examples of inorganic esters include nitrates. More preferred examples of the hydrophobic cellulose derivative include those selected from the group consisting of methyl cellulose, ethyl cellulose, hydroxy propyl cellulose, cellulose acetate, cellulose propionate, cellulose butyrate, cellulose nitrate, and combinations thereof. In this context, "combinations" refers to mixtures and copolymers thereof. The mixtures and copolymers can include one or more members of the group and/or other monomers/polymers. Examples of copolymers include hydroxypropyl methyl cellulose, hydroxypropyl ethyl cellulose, methyl ethyl cellulose, cellulose acetate propionate, cellulose acetate butyrate, cellulose propionate butyrate, cellulose acetate propionate butyrate, and the like. Particularly preferred hydrophobic cellulose derivatives include cellulose acetate butyrate and cellulose acetate propionate.

A preferred hydrophobic cellulose derivative includes organic esters or ethers wherein the number of hydroxyl groups is from 0 to 3 per repeat unit. More preferably, the number of hydroxyl groups is from 0 to 0.5 per repeat unit, and most preferably, zero (0).

Preferably, higher molecular weights of polymers are desirable for better mechanical properties; however, the molecular weights should not be so high such that the polymer is not soluble in a processing solvent for preferred solvent-coating techniques or not miscible with the other polymer(s) in the blend. A preferred hydrophobic cellulose derivative has a number average molecular weight of at least about 10,000 g/mol, and more preferably at least about 20,000 g/mol. A preferred hydrophobic cellulose derivative has a number average molecular weight of no greater than about 200,000 g/mol, and more preferably no greater than about 100,000 g/mol, and most preferably no greater than about 70,000 g/mol.

A miscible polymer blend of the present invention includes a polyvinyl homopolymer or copolymer. Herein, a "copolymer" includes two or more different repeat units, thereby encompassing terpolymers, tetrapolymers, and the like. A polyvinyl homopolymer or copolymer is preferably present in the miscible polymer blend in an amount of at least about 0.1 wt-%, and more preferably up to about 99.9 wt-%, based on the total weight of the blend, depending on the active agent and specific choice of polymers.

The polyvinyl homopolymer or copolymer is preferably selected from the group consisting of a polyvinyl alkylate, a polyvinyl alkyl ether, a polyvinyl acetal, and combinations thereof. In this context, "combinations" refers to mixtures and copolymers thereof. The copolymers can include one or more members of the group and/or other monomers/polymers. Thus, polyvinyl copolymers include copolymers of vinyl alkylates, vinyl alkyl ethers, and vinyl acetals with each other and/or with a variety of other monomers including styrene, hydrogenated styrene, (meth)acrylates (i.e., esters of acrylic acid or methacrylic acid also referred to as acrylates and methacrylates, including alkyl and/or aryl (meth) acrylates), cyanoacrylates (i.e., esters of cyanoacrylic acid including alkyl and/or aryl cyanoacrylates), and acrylonitrile.

Preferred polyvinyl homopolymers or copolymers thereof include polyvinyl formal, polyvinyl butyral, polyvinyl ether, polyvinyl acetate, polyvinyl propionate, polyvinyl butyrate, and combinations thereof (i.e., mixtures and copolymers thereof). A particularly preferred polyvinyl homopolymer or copolymer is a homopolymer or copolymer of polyvinyl alkylates including, for example, polyvinyl acetate, polyvinyl propionate, or polyvinyl butyrate. Of these, polyvinyl acetate is particularly desirable.

Preferably, higher molecular weights of polymers are desirable for better mechanical properties; however, the molecular weights should not be so high such that the polymer is not soluble in a processing solvent for preferred solvent-coating techniques or not miscible with the other polymer(s) in the blend. A preferred hydrophobic polyvinyl homopolymer or copolymer has a number average molecular weight of at least about 10,000 g/mol, and more preferably at least about 50,000 g/mol. A preferred hydrophobic polyvinyl homopolymer or copolymer has a weight average molecular weight of no greater than about 1,000,000 g/mol, and more preferably no greater than about 200,000 g/mol.

Preferably, the polyvinyl homopolymer or copolymer has a lower glass transition temperature (Tg) than the hydrophobic cellulose derivative. For example, a preferred combination includes cellulose acetate butyrate, which has a Tg of 100-120°C, and polyvinyl acetate, which has a Tg of 20-30°C. By combining such high and low Tg polymers, the active agent delivery system can be tuned for the desired dissolution time of the active agent.

Preferably, at least one of the following is true: the difference between the solubility parameter of the active agent and the solubility parameter of the hydrophobic cellulose derivative is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$); and the difference between the solubility parameter of the active agent and at least one solubility parameter of the polyvinyl homopolymer or copolymer is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$). More preferably, both of these statements are true. Preferably, the difference between the solubility parameter of the hydrophobic cellulose derivative and the polyvinyl homopolymer or copolymer is no greater than about 5 $J^{1/2}/cm^{3/2}$ (preferably, no greater than about 3 $J^{1/2}/cm^{3/2}$).

For example, the preferred polymers, cellulose acetate butyrate and polyvinyl acetate, have solubility parameters of 22 $J^{1/2}/cm^{3/2}$ and 21 $J^{1/2}/cm^{3/2}$, respectively. Such values were obtained as described below in Table 1. This blend can be used with active agents such as dexamethasone, which has a solubility parameter of 27 $J^{1/2}/cm^{3/2}$ based on Hoftyzer and van Kevelen's method and Hoy's method (See Note 2 of Table 1) and 21.1 $J^{1/2}/cm^{3/2}$ based on the molecular dynamics simulation (See Note 3 of Table 1).

Table 1

| Polymers | Solubility parameter ($J^{1/2}/cm^{3/2}$) | Source | Notes | Tg (°C) | Notes | Source |
|---|---|---|---|---|---|---|
| poly(vinyl acetate) | 20.85 | 1 | | 28 | | 1 |
| cellulose acetate butyrate (acetyl 29.5 wt-%, butyryl 17 wt-%) | 21.8 | 2 | The total numbers of acetyl, butyryl, and OH has to be 3 per repeat unit. It was estimated the wt-% of OH was 1.1 and the molecular weight of the repeat unit was 303 g/mol. Fedors volume 188 $cm^3$/mol | 110 | | TSC |
| dexamethasone | 27.25 | 2 | All rings were treated as aliphatic. Hydroxyl groups were not involved in hydrogen bonding. Fedors volume 205 $cm^3$/mol | | | |

(continued)

| Polymers | Solubility parameter $(J^{1/2}/cm^{3/2})$ | Source | Notes | Tg (°C) | Notes | Source |
|---|---|---|---|---|---|---|
|  | 21.1 | 3 |  |  |  |  |

Source for Solubility Parameters:

1. D.W.van Krevelen, Properties of Polymers, 3rd ed., Elsevier, 1990. Table 7.5. Data were the average if there were two values listed in the sources.

2. Average of the calculated values based on Hoftyzer and van Kevelen's (H-vK) method (where the volumes of the chemicals were calculated based on Fedors' method) and Hoy's method. See Chapter 7, D.W.van Krevelen, Properties of Polymers, 3rd ed., Elsevier,1990, for details of all the calculations, where Table 7.8 was for Hoftyzer and van Kevelen's method, Table 7.3 for Fedors' method, and Table 7.9 and 7.10 for Hoy's method.

3. Values based on the molecular dynamics simulation with Accelrys Materials Studio, Accelrys Inc., San Diego, CA. Simulation began with building molecular models with Atomistic Tool. The atoms of the drug were assigned groused based their charges. After minimizing the energy of the molecule, amorphous cells that contained a number of molecules were built (total number of atoms of each cell was no more than 9500). Energy minimizations were conducted to eliminate any strain that occurred during the amorphous cell building. Dynamics simulations were consequently conducted for a simulated time of about 200 ps. The cohesive energy density and solubility parameter were calculated based on about 5 configurations the final stages of the simulation. COMPASS force field was used.

Source of Tg's (the reported value is the average if there are two values listed in the sources):

1. Table 6.6, M. J. He, W. X. Chen, and X. X. Dong, Polymer Physics, revised version, FuDan University Press, ShangHai, China, 2000. Data were the average if there were two values listed in the sources.

The polymers in the miscible polymer blends can be crosslinked or not. Similarly, the blended polymers can be crosslinked or not. Such crosslinking can be carried out by one of skill in the art after blending using standard techniques.

In the active agent systems of the present invention, the active agent passes through a miscible polymer blend having a "critical" dimension. This critical dimension is along the net diffusion path of the active agent and is preferably no greater than about 1000 micrometers (i.e., microns), although for shaped objects it can be up to about 10,000 microns.

For embodiments in which the miscible polymer blends form coatings or free-standing films (both generically referred to herein as "films"), the critical dimension is the thickness of the film and is preferably no greater than about 1000 microns, more preferably no greater than about 500 microns, and most preferably no greater than about 100 microns. A film can be as thin as desired (e.g., 1 nanometer), but are preferably no thinner than about 10 nanometers, more preferably no thinner than about 100 nanometers. Generally, the minimum film thickness is determined by the volume that is needed to hold the required dose of active agent and is typically only limited by the process used to form the materials. For all embodiments herein, the thickness of the film does not have to be constant or uniform. Furthermore, the thickness of the film can be used to tune the duration of time over which the active agent is released.

For embodiments in which the miscible polymer blends form shaped objects (e.g., microspheres, beads, rods, fibers, or other shaped objects), the critical dimension of the object (e.g., the diameter of a microsphere or a rod) is preferably no greater than about 10,000 microns, more preferably no greater than about 1000 microns, even more preferably no greater than about 500 microns, and most preferably no greater than about 100 microns. The objects can be as small as desired (e.g., 10 nanometers for the critical dimension). Preferably, the critical dimension is no less than about 100 microns, and more preferably no less than about 500 nanometers.

In one embodiment, the present invention provides a medical device characterized by a substrate surface overlayed with a polymeric top coat layer that includes a miscible polymer blend, preferably with a polymeric undercoat (primer) layer. When the device is in use, the miscible polymer blend is in contact with a bodily fluid, organ, or tissue of a subject.

The invention is not limited by the nature of the medical device; rather, any medical device can include the polymeric coating layer that includes the miscible polymer blend. Thus, as used herein, the term "medical device" refers generally to any device that has surfaces that can, in the ordinary course of their use and operation, contact bodily tissue, organs or fluids such as blood. Examples of medical devices include, without limitation, stents, stent grafts, anastomotic connectors, leads, needles, guide wires, catheters, sensors, surgical instruments, angioplasty balloons, wound drains, shunts, tubing, urethral inserts, pellets, implants, pumps, vascular grafts, valves, pacemakers, and the like. A medical device can be an extracorporeal device, such as a device used during surgery, which includes, for example, a blood oxygenator, blood pump, blood sensor, or tubing used to carry blood, and the like, which contact blood which is then returned to the subject. A medical device can likewise be an implantable device such as a vascular graft, stent, stent graft, anastomotic connector, electrical stimulation lead, heart valve, orthopedic device, catheter, shunt, sensor, replacement device for nucleus pulposus, cochlear or middle ear implant, intraocular lens, and the like. Implantable devices include transcutaneous devices such as drug injection ports and the like.

In general, preferred materials used to fabricate the medical device of the invention are biomaterials. A "biomaterial" is a material that is intended for implantation in the human body and/or contact with bodily fluids, tissues, organs and the like, and that has the physical properties such as strength, elasticity, permeability and flexibility required to function for the intended purpose. For implantable devices in particular, the materials used are preferably biocompatible materials, i.e., materials that are not overly toxic to cells or tissue and do not cause undue harm to the body. The invention is not limited by the nature of the substrate surface for embodiments in which the miscible polymer blends form polymeric coatings. For example, the substrate surface can be composed of ceramic, glass, metal, polymer, or any combination thereof. In embodiments having a metal substrate surface, the metal is typically iron, nickel, gold, cobalt, copper, chrome, molybdenum, titanium, tantalum, aluminum, silver, platinum, carbon, and alloys thereof. A preferred metal is stainless steel, a nickel titanium alloy, such as NITINOL, or a cobalt chrome alloy, such as NP35N.

A polymeric coating that includes a miscible polymer blend can adhere to a substrate surface by either covalent or non-covalent interactions. Non-covalent interactions include ionic interactions, hydrogen bonding, dipole interactions, hydrophobic interactions and van der Waals interactions, for example.

Preferably, the substrate surface is not activated or functionalized prior to application of the miscible polymer blend coating, although in some embodiments pretreatment of the substrate surface may be desirable to promote adhesion. For example, a polymeric undercoat layer (i.e., primer) can be used to enhance adhesion of the polymeric coating to the substrate surface. Suitable polymeric undercoat layers are disclosed in Applicants' copending U.S. Provisional Application Serial No. 60/403,479, filed on August 13, 2002, and U.S. Patent Application US 2004/039437, filed on even date herewith, both entitled MEDICAL DEVICE EXHIBITING IMPROVED ADHESION BETWEEN POLYMERIC COATING AND SUBSTRATE. A particularly preferred undercoat layer disclosed therein consists essentially of a polyurethane. Such a preferred undercoat layer includes a polymer blend that contains polymers other than polyurethane but only in amounts so small that they do not appreciably affect the durometer, durability, adhesive properties, structural integrity and elasticity of the undercoat layer compared to an undercoat layer that is exclusively polyurethane.

When a stent or other vascular prosthesis is implanted into a subject, restenosis is often observed during the period beginning shortly after injury to about four to six months later. Thus, for embodiments of the invention that include stents, the generalized dissolution rates contemplated are such that the active agent should ideally start to be released immediately after the prosthesis is secured to the lumen wall to lessen cell proliferation. The active agent should then continue to dissolute for up to about four to six months in total.

The invention is not limited by the process used to apply the polymer blends to a substrate surface to form a coating. Examples of suitable coating processes include solution processes, powder coating, melt extrusion, or vapor deposition.

A preferred method is solution coating. For solution coating processes, examples of solution processes include spray coating, dip coating, and spin coating. Typical solvents for use in a solution process include tetrahydrofuran (THF), methanol, ethanol, ethylacetate, dimethylformamide (DMF), dimethyacetamide (DMA), dimethylsulfoxide (DMSO), dioxane, N-methyl pyrollidone, chloroform, hexane, heptane, cyclohexane, toluene, formic acid, acetic acid, and/or dichloromethane. Single coats or multiple thin coats can be applied.

Similarly, the invention is not limited by the process used to form the miscible polymer blends into shaped objects. Such methods would depend on the type of shaped object. Examples of suitable processes include extrusion, molding, micromachining, emulsion polymerization methods, electrospray methods, etc.

For preferred embodiments in which the active agent delivery system includes one or more coating layers applied to a substrate surface, a preferred embodiment includes the use of a primer, which is preferably applied using a "reflow method," which is described in Applicants' copending U.S. Provisional Application Serial No. 60/403,479, filed on August 13, 2002, and U.S. Patent Application Serial US 2004/039437, filed on even date herewith, both entitled MEDICAL DEVICE EXHIBITING IMPROVED ADHESION BETWEEN POLYMERIC COATING AND SUBSTRATE.

Preferably, in this "reflow method," the device fabrication process involves first applying an undercoat polymer to a substrate surface to form the polymeric undercoat layer, followed by treating the polymeric undercoat layer to reflow the undercoat polymer, followed by applying a miscible polymer blend, preferably with an active agent incorporated therein, to the reformed undercoat layer to form a polymeric top coat layer. Reflow of the undercoat polymer can be accomplished in any convenient manner, e.g., thermal treatment, infrared treatment, ultraviolet treatment, microwave treatment, RF treatment, mechanical compression, or solvent treatment. To reflow the undercoat polymer, the undercoat layer is heated to a temperature that is at least as high as the "melt flow temperature" of the undercoat polymer, and for a time sufficient to reflow the polymer. The temperature at which the polymer enters the liquid flow state (i.e., the "melt flow temperature") is the preferred minimum temperature that is used to reflow the polymer according to the invention. Typically 1 to 10 minutes is the time period used to reflow the polymer using a thermal treatment in accordance with the invention. The melt flow temperature for a polymer is typically above the Tg (the melt temperature for a glass) and the Tm (the melt temperature of a crystal) of the polymer.

EXAMPLES

The present invention is illustrated by the following examples.

Thermal Stimulated Current (TSC) Test Method

Thermal stimulated current (TherMold Partners, L.P., Stamford, CT) was used to determine thermal transitions in PVAC/CAB blends. A piece of a film of about 1 centimeter (cm) by 1 cm was placed on the surface of a polytetrafluoroethylene (PTFE) film (about 50 microns thick). The two films were placed between the plate-pivot electrodes of the TSC. The testing chamber was purged by alternately turning on He gas (ultra high purity, Toll Gas and Welding Co., Plymouth, MN) and vacuum three times. The pressure of He was about 0.08 megapascal (MPa) to 0.12 MPa. After purging, the chamber was filled with He gas of the same pressure. The sample was heated to 200°C and a voltage of 200 Volts per millimeter (V/mm) was applied across the thickness of the sample and PTFE films. After 2 minutes, the sample was quenched to - 50°C within about 10 minutes while the 200 V/mm of electric voltage was maintained. The electric field was then turned off and the sample heated at 2°C/minute to 200°C. Electric current across the films was recorded during this heating process. The recorded current-temperature curve was used to determine thermal transitions. As the PTFE film was used between the plate electrode and the sample film, one of its thermal transition peaks from 15-25°C appeared in the TSC curves of all the samples. In order to compare the thermal transition temperatures, the current was scaled such that the highest peak of each sample was reduced to 1. Therefore, the current values in the figures were in arbitrary units.

Sample Preparation with Dexamethasone

Polyvinyl acetate (PVAC, Mw (weight average molecular weight) = 167 to 500 killograms per mole (kg/mol)) and cellulose acetate butyrate (CAB, 29.5 wt-% acetyl and 17 wt-% butyryl, Mn (number average molecular weight) = 65 kg/mol), both from Sigma-Aldrich Company, Milwaukee, WI, were dried in a vacuum oven and separately dissolved with anhydrous tetrahydrofuran (THF). The polymer concentration in both solutions was about 1 wt-%. A THF solution with 1 wt-% of dexamethasone (Sigma-Aldrich) was also made in a similar way. The three solutions were mixed in varying ratios to make 5 different samples with the compositions shown in Table 2.

Table 2

| PVAC/CAB (weight ratio) | 100/0 | 70/30 | 50/50 | 30/70 | 0/100 |
| --- | --- | --- | --- | --- | --- |
| Dexamethasone (wt-%) based on total solids | 10.8 | 10.6 | 10.1 | 10.4 | 9.7 |

Dissolution samples were prepared with stainless steel (316L) shims that were cleaned by rinsing with THF and dried. The cleaned shims were coated with a solution of 1 wt-% poly(ether urethane) (PELLETHANE 75D, Dow Chemical Co., Midland, MI) dissolved in THF. Before dissolving PELLETHANE 75D poly(ether urethane) in THF, it was dried overnight at 70°C under reduced pressure, then melted and pressed between two hot plates at 230°C for 5-10 minutes. Then the films were cooled and dissolved in anhydrous tetrahydrofuran (THF) at about 25°C by stirring with a magnetic bar overnight.

The coated shims were allowed to dry overnight under nitrogen then thermally treated at 215-220°C for 5-10 minutes. This pretreatment formed a primer on the surface of the shim to promote adhesion with polymer/active agent layers. The primed shims were coated with the solutions listed above and dried overnight under nitrogen. The shims were weighed after each step. Based on the weight difference, the total amount of polymer/active agent coating was determined as was the thickness of the coating. Typical dissolution samples had 4-5 milligrams (mg) dried coating per shim that was about 10 microns thick.

Samples for miscibility tests were made in a similar way except that there was no primer coating. Typical sample thickness was about 100 microns and there was no active agent included therein.

Miscibility

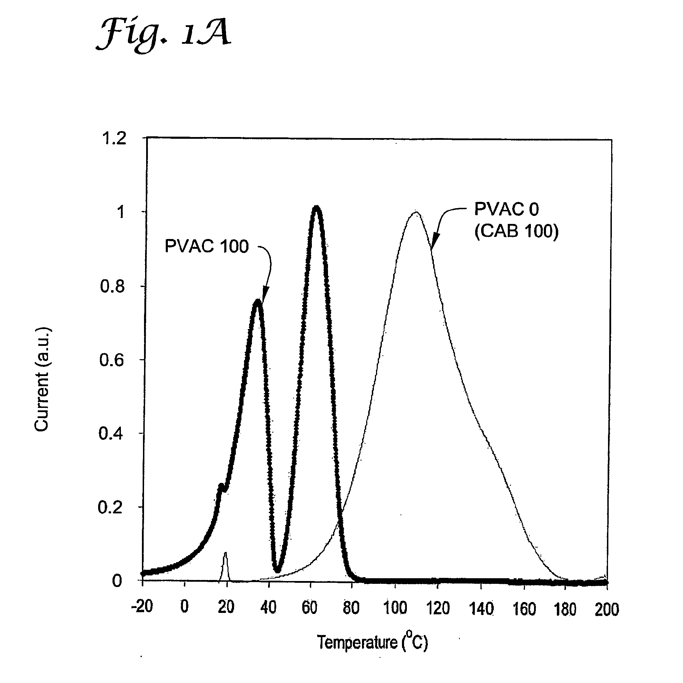

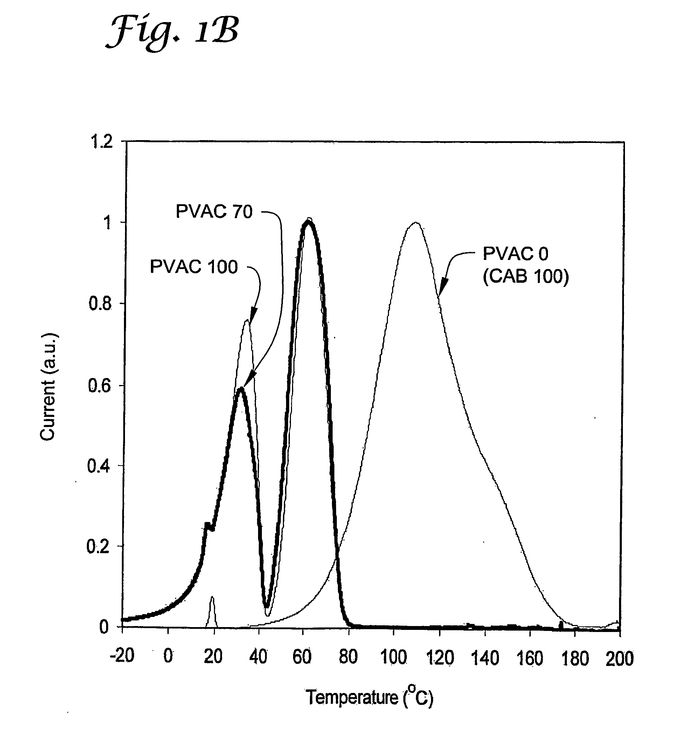

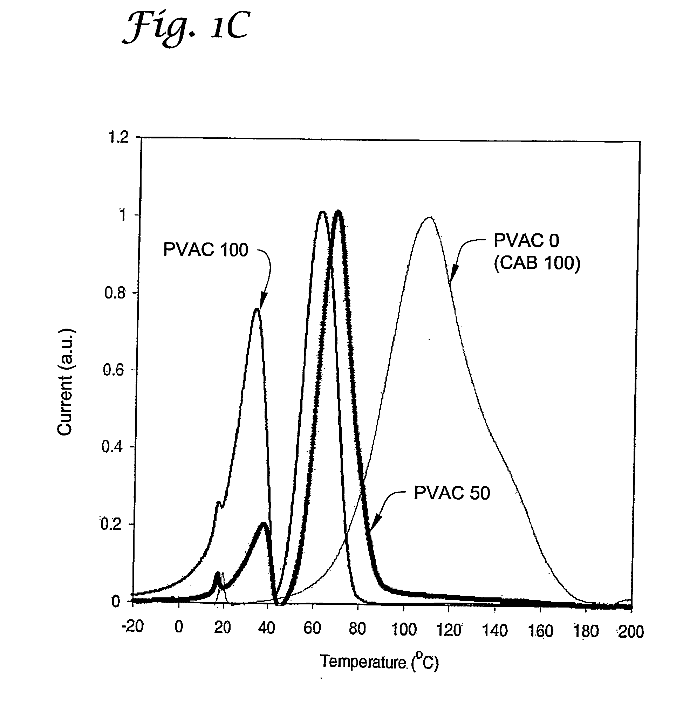

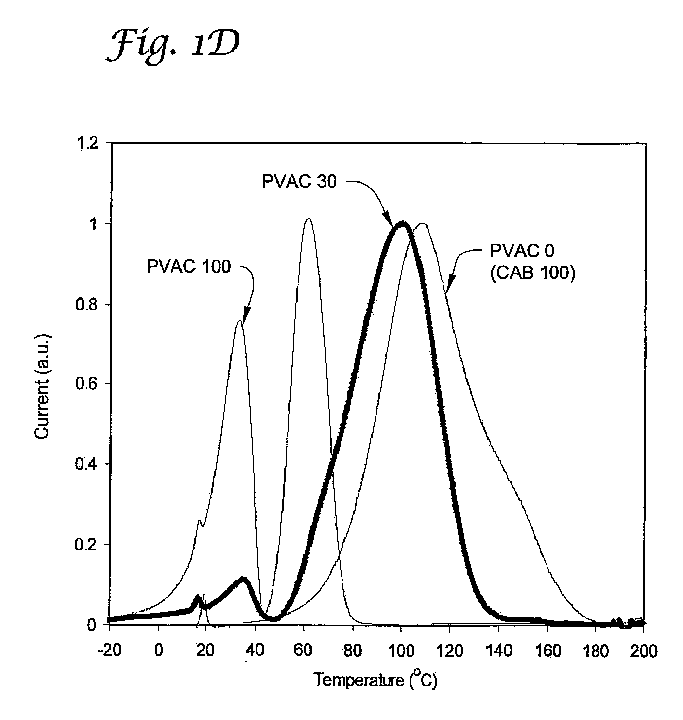

Miscibility of PVAC and CAB was tested by measuring the thermal transition temperatures of various blends. Differential scanning calorimeter (DSC), dynamic mechanical analysis (DMA), and thermally stimulated current (TCS) were used to measure the glass transition temperature (Tg) and other transitions. TSC had the strongest signals. It provided consistent results as shown in Figures 1 A-D. For the pure PVAC (Figure 1A), TSC showed two transition peaks, centered at 34°C and 62°C, respectively. Pure CAB (Figure 1 A) had one peak centered at about 110°C in its TSC curve. When 30 wt-% of CAB was blended into PVAC, neither of the transition peaks of the PVAC was changed (Figure 1 B). However, the glass transition of pure CAB disappeared, which suggests that this blend was miscible. When the amount of CAB was increased to 50 wt-%, the two transition peaks of PVAC shifted to higher temperature but no Tg peak for the pure CAB was observed (Figure 1C). This suggests that the PVAC and CAB were also miscible in 50/50 blend. In the blend containing 70 wt-% of CAB, the temperatures of the transition peaks were even higher, which once again suggests a miscible blend (Figure 1 D). All of the films were clear and transparent, supporting our conclusion that these were miscible blends.

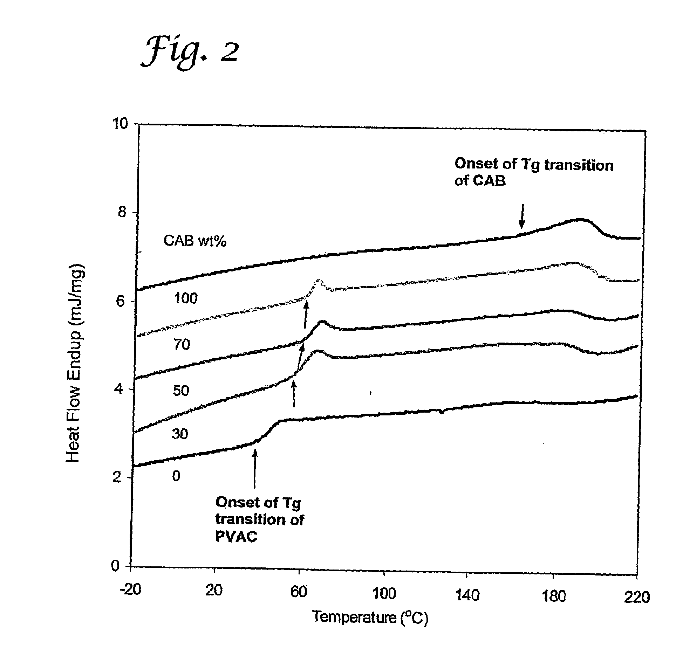

DSC analysis was conducted with PYRIS 1 DSC (PerkinElmer Company, Wellesley, MA). The scanning was programmed from -50°C to 220°C at 40°C/minutes. The sample size was about 10 milligrams (mg). As shown in Figure 2, the pure PVAC had a Tg transition at about 39°C and the pure CAB had a Tg at about 167°C. When PVAC and CAB were blended at a weight ratio of 70/30, the Tg corresponding to PVAC increased to 55°C. This suggested that the PVAC and CAB are partially miscible at this ratio. Adding more CAB, Tg corresponding to the PVAC further increased but at a slower rate. The Tg corresponding to CAB decreased upon mixing with PVAC. All these results suggested that the PVAC and CAB are partially miscible over the entire range of mixing. This result was slightly different from that based on the TSC test described above. However, the conclusion using the miscibility definition of the present invention was the same, i.e., PVAC and CAB are miscible.

Dissolution of Dexamethasone

Dissolution of dexamethasone from PVAC/CAB polymer matrix was conducted with the polymer/active agent coated shims prepared as described above. The coated shims were cut into pieces, measured, and the areas were calculated for normalization. Each piece was immersed in a vial containing 3 millimeters (mL) of phosphate buffered saline solution (PBS, potassium phosphate monobasic (NF tested), 0.144 grams per liter (g/L), sodium chloride (USP tested), 9 g/L, and sodium phosphate dibasic (USP tested) 0.795 g/L, pH = 7.0 to 7.2 at 37°C, purchased from HyClone, Logan, UT). The amount of sample and PBS solution were chosen so that the concentration of active agent would be detectable by UV-Vis spectrophotometry, yet the concentration of active agent in the sample would not exceed 5% of the solubility of active agent in PBS (sink condition) during the experiment. Approximately 2 milligrams (mg) of coating, containing about 200 micrograms of active agent, and 3 milliliters (mL) of PBS that were preheated to 37°C were used. The dissolution test was run at 37°C and the samples were agitated on a shaker at about 10 cycles per minute. The PBS was removed from the sample vials and analyzed at various times to determine the concentration of active agent in each sample. The concentration of active agent in PBS was measured with UV-Vis spectroscopy (HP 4152A) at the wavelength of 243 nanometers (nm). The concentration of active agent in each sample was calculated by comparing to a standard curve created by a serial dilution method. The cuvette was carefully cleaned after each measurement to minimize accumulation of the hydrophobic active agent on the cuvette surface. The cuvette was considered clean when the baseline was at least one order of magnitude lower than that of the measured active agent signal. The PBS was refreshed at each analysis time point.

Dissolution Data Analysis

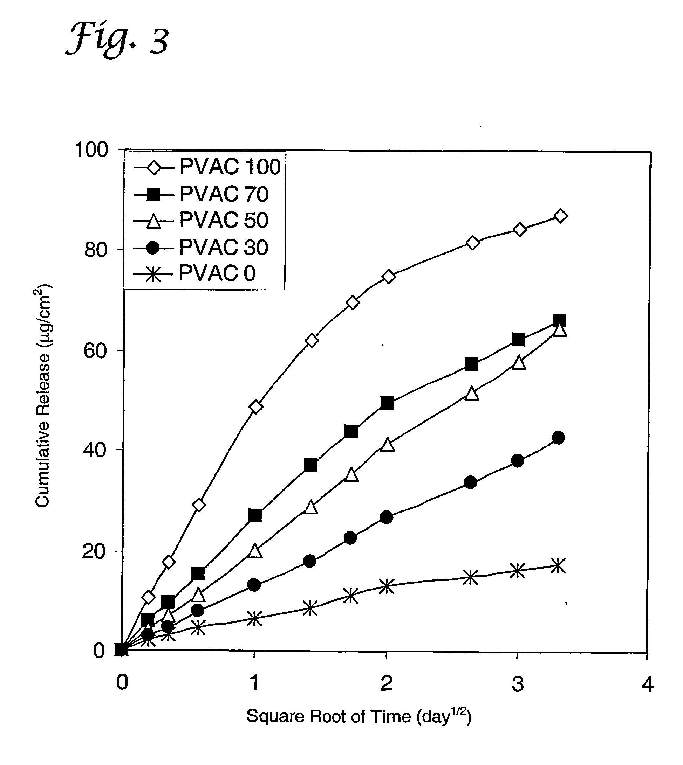

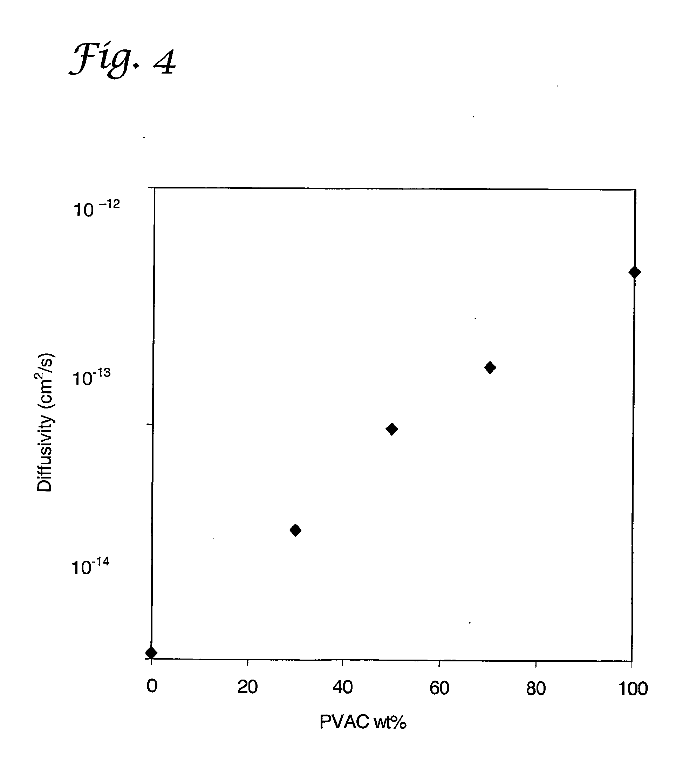

Figure 3 shows the cumulative release of dexamethasone increased with an increasing amount of PVAC in the blend. These release curves clearly show that by blending PVAC and CAB, it was possible to vary the release rate by varying the relative amounts of two homopolymers. Based on the curves, the diffusion coefficients of dexamethasone from these blends were calculated using the following equation and plotted as a function of blend composition in Figure 4.

$$D = (\frac{M_t}{4M_\infty})^2 \cdot \frac{\pi x^2}{t}$$

wherein D = diffusion coefficient; $M_t$ = cumulative release; $M_\infty$ = total loading of active agent; x = the critical dimension (e.g., thickness of the film); and t = the dissolution time.

The log of the diffusion coefficient was a linear function of the blend composition, demonstrating that the active agent release rate can be tuned by using miscible polymer blends. Additionally, the data presented in Figure 3 shows no burst, which indicates that the release of the active agent was predominantly under permeation control.

The invention is not limited to the exact details shown and described; many variations will be apparent to one skilled in the art and are intended to be included within the invention defined by the claims.

**Claims**

1. An active agent delivery system comprising an active agent and a miscible polymer blend comprising a hydrophobic cellulose derivative and a polyvinyl homopolymer or copolymer selected from the group consisting of a polyvinyl alkylate homopolymer or copolymer, a polyvinyl alkyl ether homopolymer or copolymer, a polyvinyl acetal homopolymer or copolymer, and combinations thereof.

2. The system of claim 1 wherein the active agent is incorporated within the miscible polymer blend.

3. The system of claim 2 wherein the active agent is present within the miscible polymer blend in an amount of 0.1 wt-% to 80 wt-%, based on the total weight of the miscible polymer blend and the active agent.

4. The system of claim 1 wherein the miscible polymer blend initially provides a barrier to permeation of the active agent.

5. The system of claim 4 wherein the active agent is incorporated within an inner matrix.

6. The system of claim 5 wherein the active agent is present within the inner matrix in an amount of 0.1 wt-% to 100 wt-%, based on the total weight of the inner matrix including the active agent.

7. The system of claim 1 wherein:

    each of the active agent, the hydrophobic cellulose derivative, and the polyvinyl homopolymer or copolymer has a solubility parameter; and
    at least one of the following relationships is true:

       the difference between the solubility parameter of the active agent and the solubility parameter of the hydrophobic cellulose derivative is no greater than 10 $J^{1/2}/cm^{3/2}$; and
       the difference between the solubility parameter of the active agent and at least one solubility parameter of the polyvinyl homopolymer or copolymer is no greater than 10 $J^{1/2}/cm^{3/2}$.

8. The system of claim 7 wherein the active agent has a solubility parameter within at least 10 $J^{1/2}/cm^{3/2}$ of the solubility parameters of each of cellulose acetate butyrate and polyvinyl acetate.

9. The system of claim 1 wherein:

    each of the hydrophobic cellulose derivative and the polyvinyl homopolymer or copolymer has a solubility parameter; and
    the difference between the solubility parameter of the hydrophobic cellulose derivative and at least one solubility parameter of the polyvinyl homopolymer or copolymer is no greater than 5 $J^{1/2}/cm^{3/2}$.

10. The system of claim 1 wherein the hydrophobic cellulose derivative is selected from the group consisting of methyl cellulose, ethyl cellulose, hydroxy propyl cellulose, cellulose acetate, cellulose propionate, cellulose butyrate, cellulose nitrate, and combinations thereof.

11. The system of claim 1 wherein the polyvinyl homopolymer or copolymer is a polyvinyl alkylate homopolymer or copolymer.

12. The system of claim 11 wherein the polyvinyl alkylate homopolymer or copolymer is a homopolymer or copolymer of polyvinyl acetate, polyvinyl propionate, or polyvinyl butyrate.

13. The system of claim 11 wherein the polyvinyl alkylate homopolymer or copolymer is a polyvinyl acetate homopolymer or copolymer.

14. The system of claim 1 wherein the active agent is hydrophobic and has a molecular weight of no greater than 1200 g/mol.

15. The system of claim 1 wherein the hydrophobic cellulose derivative is present in the miscible polymer blend in an amount of 0.1 wt-% to 99.9 wt-%, based on the total weight of the blend.

16. The system of claim 1 wherein the polyvinyl homopolymer or copolymer is present in the miscible polymer blend in an amount of 0.1 wt-% to 99.9 wt-%, based on the total weight of the blend.

17. The system of claim 1 which is in the form of microspheres, beads, rods, fibers, or other shaped objects.

18. The system of claim 17 wherein the critical dimension of the object is no greater than 10,000 microns.

19. The system of claim 1 which is in the form of a film.

20. The system of claim 19 wherein the thickness of the film is no greater than 1000 microns.

21. The system of claim 19 wherein the film forms a patch or a coating on a surface.

22. The active agent delivery system of claim 1 wherein

the active agent is hydrophobic and has a molecular weight of no greater than 1200 g/mol;
each of the active agent, the hydrophobic cellulose derivative, and the polyvinyl homopolymer or copolymer has a solubility parameter;
the difference between the solubility parameter of the active agent and the solubility parameter of the hydrophobic cellulose derivative is no greater than 10 $J^{1/2}/cm^{3/2}$, and the difference between the solubility parameter of the active agent and at least one solubility parameter of the polyvinyl homopolymer or copolymer thereof is no greater than 10 $J^{1/2}/cm^{3/2}$; and
the difference between the solubility parameter of the hydrophobic cellulose derivative and at least one solubility parameter of the polyvinyl homopolymer or copolymer thereof is no greater than 5 $J^{1/2}/cm^{3/2}$.

23. The active agent delivery system of claim 1 wherein delivery of the active agent occurs predominantly under permeation control.

24. A medical device comprising the active agent delivery system of claim 1.

25. A medical device comprising the active agent delivery system of claim 22.

26. A medical device comprising the active agent delivery system of claim 23.

27. A medical device comprising:

a substrate surface;
a polymeric undercoat layer adhered to the substrate surface; and
a polymeric top coat layer adhered to the polymeric undercoat layer;
wherein the polymeric top coat layer comprises the system of claim 2.

28. The medical device of claim 27 wherein the polymer undercoat layer comprises a polyurethane.

29. The medical device of claim 27 which is an implantable device.

30. The medical device of claim 27 which is an extracorporeal device.

31. The medical device of claim 27 selected from the group consisting of a stent, stent graft, anastomotic connector, lead, needle, guide wire, catheter, sensor, surgical instrument, angioplasty balloon, wound drain, shunt, tubing, urethral insert, pellet, implant, blood oxygenator, pump, vascular graft, valve, pacemaker, orthopedic device, replacement device for nucleus pulposus, and intraocular lense.

32. The medical device of claim 27 wherein the active agent is hydrophobic and has a molecular weight of no greater than 1200 g/mol.

33. The medical device of claim 27 wherein delivery of the active agent occurs predominantly under permeation control.

34. A stent comprising:

a substrate surface;
a polymeric undercoat layer adhered to the substrate surface; and
a polymeric top coat layer adhered to the undercoat layer;

wherein the polymeric top coat layer comprises the system of claim 2.

**35.** The stent of claim 34 wherein the active agent is hydrophobic and has a molecular weight of no greater than 1200 g/mol.

**36.** The medical device of claim 34 wherein delivery of the active agent occurs predominantly under permeation control.

**37.** A method of forming an active agent delivery system comprising:

combining a hydrophobic cellulose derivative and a polyvinyl homopolymer or copolymer to form a miscible polymer blend, wherein the polyvinyl homopolymer or copolymer is selected from the group consisting of a polyvinyl alkylate homopolymer or copolymer, a polyvinyl alkyl ether homopolymer or copolymer, a polyvinyl acetal homopolymer or copolymer, and combinations thereof; and
combining an active agent with the miscible polymer blend.

**38.** The method of claim 37 wherein the active agent is incorporated within the miscible polymer blend.

**39.** The method of claim 37 wherein the active agent is incorporated within an inner matrix and the miscible polymer blend initially provides a barrier to permeation of the active agent.

**40.** The method of claim 37 wherein the active agent is hydrophobic and has a molecular weight of no greater than 1200 g/mol.

**Patentansprüche**

**1.** Ein System zur Abgabe von Wirkstoffen, enthaltend einen Wirkstoff und ein Blend mischbarer Polymere, das ein hydrophobes Cellulosederivat und ein Polyvinyl-Homopolymer oder -Copolymer enthält, das ausgewählt ist aus der Gruppe bestehend aus einem Polyvinylalkylat-Homopolymer oder -Copolymer, einem Polyvinylalkylether-Homopolymer oder -Copolymer, einem Polyvinylacetal-Homopolymer oder -Copolymer und Mischungen daraus.

**2.** Das System gemäß Anspruch 1, bei dem der Wirkstoff in dem Blend mischbarer Polymere enthalten ist.

**3.** Das System gemäß Anspruch 2, bei dem der Wirkstoff in einer Menge von 0,1 Gew.-% bis 80 Gew.-%, bezogen auf das Gesamtgewicht des Blends mischbarer Polymere und des Wirkstoffs, in dem Blend mischbarer Polymere enthalten ist.

**4.** Das System gemäß Anspruch 1, bei dem das Blend mischbarer Polymere anfänglich eine Barriere gegen die Permeation des Wirkstoffs bildet.

**5.** Das System gemäß Anspruch 4, bei dem der Wirkstoff innerhalb einer inneren Matrix eingeschlossen ist.

**6.** Das System gemäß Anspruch 5, bei dem der Wirkstoff in der inneren Matrix in einer Menge von 0,1 Gew.-% bis 100 Gew.-%, bezogen auf das Gesamtgewicht der inneren Matrix einschließlich des Wirkstoffs, enthalten ist.

**7.** Das System gemäß Anspruch 1, worin:

der Wirkstoff, das hydrophobe Cellulosederivat und das Polyvinyl-Homopolymer oder -Copolymer jeweils einen Löslichkeitsparameter aufweisen; und
mindestens eine der folgenden Beziehungen zutrifft:

die Differenz zwischen dem Löslichkeitsparameter des Wirkstoffs und dem Löslichkeitsparameter des hydrophoben Cellulosederivats ist nicht größer als 10 $J^{1/2}/cm^{3/2}$; und
die Differenz zwischen dem Löslichkeitsparameter des Wirkstoffs und mindestens einem Löslichkeitsparameter des Polyvinyl-Homopolymers oder -Copolymers ist nicht größer als 10 J$^{1/2}$/cm$^{3/2}$.

8.  Das System gemäß Anspruch 7, bei dem der Wirkstoff einen Löslichkeitsparameter aufweist, der innerhalb eines Bereichs von mindestens 10 J$^{1/2}$/cm$^{3/2}$ um die jeweiligen Löslichkeitsparameter von Celluloseacetatbutyrat und Polyvinylacetat liegt.

9.  Das System gemäß Anspruch 1, bei dem:

    das hydrophobe Cellulosederivat und das Polyvinyl-Homopolymer oder -Copolymer jeweils einen Löslichkeitsparameter aufweisen; und
    die Differenz zwischen dem Löslichkeitsparameter des hydrophoben Cellulosederivats und mindestens einem Löslichkeitsparameter des Polyvinyl-Homopolymers oder -Copolymers nicht größer als 5 J$^{1/2}$/cm$^{3/2}$ ist.

10. Das System gemäß Anspruch 1, bei dem das hydrophobe Cellulosederivat ausgewählt ist aus der Gruppe bestehend aus Methylcellulose, Ethylcellulose, Hydroxypropylcellulose, Celluloseacetat, Cellulosepropionat, Cellulosebutyrat, Cellulosenitrat, und Kombinationen davon.

11. Das System gemäß Anspruch 1, bei dem das Polyvinyl-Homopolymer oder -Copolymer ein Polyvinylalkylat-Homopolymer oder -Copolymer ist.

12. Das System gemäß Anspruch 11, bei dem das Polyvinylalkylat-Homopolymer oder -Copolymer ein Homopolymer oder -Copolymer von Polyvinylacetat, Polyvinylpropionat oder Polyvinylbutyrat ist.

13. Das System gemäß Anspruch 11, bei dem das Polyvinylalkylat-Homopolymer oder -Copolymer ein Polyvinylacetat-Homopolymer oder -Copolymer ist.

14. Das System gemäß Anspruch 1, bei dem der Wirkstoff hydrophob ist und ein Molekulargewicht von nicht mehr als 1200 g/mol aufweist.

15. Das System gemäß Anspruch 1, bei dem das hydrophobe Cellulosederivat in dem Blend mischbarer Polymere in einer Menge von 0,1 Gew.-% bis 99,9 Gew.-%, bezogen auf das Gesamtgewicht des Blends, enthalten ist.

16. Das System gemäß Anspruch 1, bei dem das Polyvinyl-Homopolymer oder -Copolymer in dem Blend mischbarer Polymere in einer Menge von 0,1 Gew.-% bis 99,9 Gew.-%, bezogen auf das Gesamtgewicht des Blends, enthalten ist.

17. Das System gemäß Anspruch 1, das in Form von Mikrokügelchen, Perlen, Stäben, Fasern oder anderen geformten Objekten vorliegt.

18. Das System gemäß Anspruch 17, bei dem die kritische Abmessung des Objekts nicht mehr als 10000 Mikronen beträgt.

19. Das System gemäß Anspruch 1 ist in Form eines Films.

20. Das System gemäß Anspruch 19, bei dem die Dicke des Films nicht mehr als 1000 Mikronen beträgt.

21. Das System gemäß Anspruch 19, bei dem der Film ein Segment oder eine Beschichtung auf einer Oberfläche bildet.

22. Das System zur Abgabe von Wirkstoffen gemäß Anspruch 1, bei dem

    der Wirkstoff hydrophob ist und ein Molekulargewicht von nicht mehr als 1200 g/mol aufweist;
    der Wirkstoff, das hydrophobe Cellulosederivat und das Polyvinyl-Homopolymer oder -Copolymer jeweils einen Löslichkeitsparameter aufweisen;
    die Differenz zwischen dem Löslichkeitsparameter des Wirkstoffs und dem Löslichkeitsparameter des hydrophoben Cellulosederivats nicht größer als 10 J$^{1/2}$/cm$^{3/2}$ ist, und die Differenz zwischen dem Löslichkeitsparameter des Wirkstoffs und mindestens einem Löslichkeitsparameter des Polyvinyl-Homopolymers oder -Copolymers davon nicht größer als 10 J$^{1/2}$/cm$^{3/2}$ ist; und
    die Differenz zwischen dem Löslichkeitsparameter des hydrophoben Cellulosederivats und mindestens einem Löslichkeitsparameter des Polyvinyl-Homopolymers oder -Copolymers davon nicht größer als 5 J$^{1/2}$ / cm$^{3/2}$ ist.

23. Das System zur Abgabe von Wirkstoffen gemäß Anspruch 1, bei dem die Abgabe des Wirkstoffs vorwiegend unter Diffusionskontrolle erfolgt.

24. Eine medizinische Vorrichtung, die das System zur Abgabe von Wirkstoffen gemäß Anspruch 1 umfasst.

25. Eine medizinische Vorrichtung, die das System zur Abgabe von Wirkstoffen gemäß Anspruch 22 umfasst.

26. Eine medizinische Vorrichtung, die das System zur Abgabe von Wirkstoffen gemäß Anspruch 23 umfasst.

27. Eine medizinische Vorrichtung, enthaltend:

   eine Substratoberfläche;
   eine polymere Grundierungsschicht, die auf der Substratoberfläche anhaftet; und
   eine polymere Deckschicht, die auf der polymeren Grundierungsschicht anhaftet;

   wobei die polymere Deckschicht das System gemäß Anspruch 2 umfasst.

28. Die medizinische Vorrichtung gemäß Anspruch 27, bei der die polymere Grundierungsschicht ein Polyurethan enthält.

29. Die medizinische Vorrichtung gemäß Anspruch 27, die eine implantierbare Vorrichtung ist.

30. Die medizinische Vorrichtung gemäß Anspruch 27, die eine extrakorporale Vorrichtung ist

31. Die medizinische Vorrichtung gemäß Anspruch 27, ausgewählt aus der Gruppe bestehend aus einem Stent, einem endoluminalen Gefäßtransplantat, einem anastomotischen Verbindungsstück, einer Leitung, einer Nadel, einem Führungsdraht, einem Katheter, einem Sensor, einem chirurgischen Instrument, einem Angioplastie-Ballon, einer Wunddrainage, einem Shunt, einem Schlauchmaterial, einem Harnröhreneinsatz, einem Pellet, einem Implantat, einem Blutoxygenator, einer Pumpe, einem vaskulären Implantat, einem Ventil bzw. einer Klappe, einem Schrittmacher, einer orthopädischen Vorrichtung, einer Vorrichtung zum Ersatz von Bandscheiben, und einer intraokularen Linse.

32. Die medizinische Vorrichtung gemäß Anspruch 27, bei der der Wirkstoff hydrophob ist und ein Molekulargewicht von nicht mehr als 1200 g/mol aufweist.

33. Die medizinische Vorrichtung gemäß Anspruch 27, bei der die Abgabe des Wirkstoffs vorwiegend unter Diffusionskontrolle erfolgt.

34. Ein Stent, enthaltend:

   eine Substratoberfläche;
   eine polymere Grundierungsschicht, die auf der Substratoberfläche anhaftet; und
   eine polymere Deckschicht, die auf der polymeren Grundierungsschicht anhaftet;

   wobei die polymere Deckschicht das System gemäß Anspruch 2 umfasst.

35. Der Stent gemäß Anspruch 34, bei dem der Wirkstoff hydrophob ist und ein Molekulargewicht von nicht mehr als 1200 g/mol aufweist.

36. Die medizinische Vorrichtung gemäß Anspruch 34, bei der die Abgabe des Wirkstoffs vorwiegend unter Diffusionskontrolle erfolgt.

37. Ein Verfahren zur Herstellung eines Systems zur Abgabe von Wirkstoffen, umfassend:

   das Vereinigen eines hydrophoben Cellulosederivats und eines Polyvinyl-Homopolymers oder -Copolymers, um ein Blend aus mischbaren Polymeren zu bilden, wobei das Polyvinyl-Homopolymer oder -Copolymer ausgewählt ist aus der Gruppe bestehend aus einem Polyvinylalkylat-Homopolymer oder -Copolymer, einem Polyvinylalkylether-Homopolymer oder -Copolymer, einem Polyvinylacetal-Homopolymer oder -Copolymer und Mischungen daraus; und

das Vereinigen eines Wirkstoffs mit dem Blend mischbarer Polymere.

**38.** Das Verfahren gemäß Anspruch 37, bei dem der Wirkstoff innerhalb des Blends mischbarer Polymere eingeschlossen wird.

**39.** Das Verfahren gemäß Anspruch 37, bei dem der Wirkstoff innerhalb einer inneren Matrix eingeschlossen wird und das Blend mischbarer Polymere anfänglich eine Barriere gegen die Permeation des Wirkstoffs bildet.

**40.** Das Verfahren gemäß Anspruch 37, bei dem der Wirkstoff hydrophob ist und ein Molekulargewicht von nicht mehr als 1200 g/mol aufweist.

**Revendications**

**1.** Système d'administration de principe actif comprenant un principe actif et un mélange de polymères miscibles comprenant un dérivé cellulosique hydrophobe et un homopolymère ou un copolymère polyvinylique choisi dans le groupe constitué par un homopolymère ou un copolymère poly(alkylate de vinyle), un homopolymère ou un copolymère poly(alkyléther de vinyle), un homopolymère ou un copolymère poly(acétal de vinyle), et des combinaisons de ceux-ci.

**2.** Système de la revendication 1, dans lequel le principe actif est incorporé dans le mélange de polymères miscibles.

**3.** Système de la revendication 2, dans lequel le principe actif est présent dans le mélange de polymères miscibles en une quantité de 0,1% en poids à 80% en poids, sur la base du poids total du mélange de polymères miscibles et du principe actif.

**4.** Système de la revendication 1, dans lequel le mélange de polymères miscibles fournit initialement une barrière à la perméation du principe actif.

**5.** Système de la revendication 4, dans lequel le principe actif est incorporé dans une matrice interne.

**6.** Système de la revendication 5, dans lequel le principe actif est présent dans la matrice interne en une quantité de 0,1% en poids à 100% en poids, sur la base du poids total de la matrice interne comprenant le principe actif.

**7.** Système de la revendication 1, dans lequel :

chacun des composés parmi le principe actif, le dérivé cellulosique hydrophobe, et l'homopolymère ou le copolymère polyvinylique a un paramètre de solubilité ; et
au moins une des relations suivantes est vraie :

la différence entre le paramètre de solubilité du principe actif et le paramètre de solubilité du dérivé cellulosique hydrophobe n'est pas supérieure à 10 $J^{1/2}/cm^{3/2}$ ; et
la différence entre le paramètre de solubilité du principe actif et au moins un paramètre de solubilité de l'homopolymère ou du copolymère polyvinylique n'est pas supérieure à 10 $J^{1/2}/cm^{3/2}$.

**8.** Système de la revendication 7, dans lequel le principe actif a un paramètre de solubilité d'au moins 10 $J^{1/2}/cm^{3/2}$ par rapport aux paramètres de solubilité de chacun des composés parmi l'acétate butyrate de cellulose et le poly(acétate de vinyle).

**9.** Système de la revendication 1, dans lequel :

chacun des composés parmi le dérivé cellulosique hydrophobe et l'homopolymère ou le copolymère polyvinylique possède un paramètre de solubilité ; et
la différence entre le paramètre de solubilité du dérivé cellulosique hydrophobe et au moins un paramètre de solubilité de l'homopolymère ou du copolymère polyvinylique n'est pas supérieure à 5 $J^{1/2}/cm^{3/2}$.

**10.** Système de la revendication 1, dans lequel le dérivé cellulosique hydrophobe est choisi dans le groupe constitué par la méthylcellulose, l'éthylcellulose, l'hydroxypropylcellulose, l'acétate de cellulose, le propionate de cellulose, le butyrate de cellulose, le nitrate de cellulose, et des combinaisons de ceux-ci.

**11.** Système de la revendication 1, dans lequel l'homopolymère ou le copolymère polyvinylique est un homopolymère ou un copolymère poly(alkylate de vinyle).

**12.** Système de la revendication 11, dans lequel l'homopolymère ou le copolymère poly(alkylate de vinyle) est un homopolymère ou un copolymère poly(acétate de vinyle), poly(propionate de vinyle), ou poly(butyrate de vinyle).

**13.** Système de la revendication 11, dans lequel l'homopolymère ou le copolymère poly(alkylate de vinyle) est un homopolymère ou un copolymère poly(acétate de vinyle).

**14.** Système de la revendication 1, dans lequel le principe actif est hydrophobe et a un poids moléculaire non supérieur à 1200 g/mol.

**15.** Système de la revendication 1, dans lequel le dérivé cellulosique hydrophobe est présent dans le mélange de polymères miscibles en une quantité de 0,1% en poids à 99,9% en poids, sur la base du poids total du mélange.

**16.** Système de la revendication 1, dans lequel l'homopolymère ou le copolymère polyvinylique est présent dans le mélange de polymères miscibles en une quantité de 0, 1 % en poids à 99,9% en poids, sur la base du poids total du mélange.

**17.** Système de la revendication 1 qui est sous forme de microsphères, de billes, de bâtonnets, de fibres, ou d'autres d'objets mis en forme.

**18.** Système de la revendication 17, dans lequel la dimension critique de l'objet n'est pas supérieure à 10 000 micromètres.

**19.** Système de la revendication 1 qui est sous forme de film.

**20.** Système de la revendication 19, dans lequel l'épaisseur du film n'est pas supérieure à 1000 micromètres.

**21.** Système de la revendication 19, dans lequel le film forme un patch ou un revêtement sur une surface.

**22.** Système d'administration de principe actif de la revendication 1, dans lequel:

le principe actif est hydrophobe et a un poids moléculaire non supérieur à 1200 g/mol ;
chacun des composés parmi le principe actif, le dérivé cellulosique hydrophobe, et l'homopolymère ou le copolymère polyvinylique a un paramètre de solubilité ;
la différence entre le paramètre de solubilité du principe actif et le paramètre de solubilité du dérivé cellulosique hydrophobe n'est pas supérieure à 10 $J^{1/2}/cm^{3/2}$, et la différence entre le paramètre de solubilité du principe actif et au moins un paramètre de solubilité de l'homopolymère polyvinylique ou d'un copolymère de celui-ci n'est pas supérieure à 10 $J^{1/2}/cm^{3/2}$; et
la différence entre le paramètre de solubilité du dérivé cellulosique hydrophobe et au moins un paramètre de solubilité de l'homopolymère polyvinylique ou d'un copolymère de celui-ci n'est pas supérieure à 5 $J^{1/2}/cm^{3/2}$.

**23.** Système d'administration de principe actif de la revendication 1, dans lequel l'administration du principe actif se produit d'une manière prédominante sous régulation de la perméation.

**24.** Dispositif médical comprenant le système d'administration de principe actif de la revendication 1.

**25.** Dispositif médical comprenant le système d'administration de principe actif de la revendication 22.

**26.** Dispositif médical comprenant le système d'administration de principe actif de la revendication 23.

**27.** Dispositif médical comprenant :

une surface substrat ;
une sous-couche de revêtement polymère adhérant à la surface substrat ; et
une couche supérieure de revêtement polymère adhérant à la sous-couche de revêtement polymère ;

dans lequel la couche supérieure de revêtement polymère comprend le système de la revendication 2.

28. Dispositif médical de la revendication 27, dans lequel la sous-couche de revêtement polymère comprend un polyuréthane.

29. Dispositif médical de la revendication 27 qui est un dispositif implantable.

30. Dispositif médical de la revendication 27 qui est un dispositif extracorporel.

31. Dispositif médical de la revendication 27 choisi dans le groupe constitué par une endoprothèse vasculaire, une prépartion par endoprothèse, un raccord d'anastomose, une dérivation, une aiguille, un fil-guide, un cathéter, un capteur, un instrument chirurgical, un ballon d'angioplastie, un drain pour plaie, un shunt, une tubulure, un manchon urétral, un pellet, un implant, un oxygénateur sanguin, une pompe, une greffe vasculaire, une valve, un pacemaker, un dispositif orthopédique, un dispositif de remplacement pour nucleus pulposus, et une lentille intra-occulaire.

32. Dispositif médical de la revendication 27, dans lequel le principe actif est hydrophobe et a un poids moléculaire non supérieur à 1200 g/mol.

33. Dispositif médical de la revendication 27, dans lequel l'administration du principe actif se produit de manière prédominante sous régulation de la perméation.

34. Endoprothèse vasculaire comprenant :

une surface de substrat ;
une sous-couche de revêtement polymère adhérant à la surface de substrat ; et
une couche supérieure de revêtement polymère adhérant à la sous-couche de revêtement ;

dans lequel la couche supérieure de revêtement polymère comprend le système de la revendication 2.

35. Endoprothèse vasculaire de la revendication 34, dans laquelle le principe actif est hydrophobe et a un poids moléculaire non supérieur à 1200 g/mol.

36. Dispositif médical de la revendication 34, dans lequel l'administration du principe actif se produit de manière prédominante sous régulation de la perméation.

37. Procédé de préparation d'un système d'administration de principe actif comprenant :

l'association d'un dérivé cellulosique hydrophobe et d'un homopolymère ou d'un copolymère polyvinylique pour former un mélange de polymères miscibles, dans lequel l'homopolymère ou le copolymère polyvinylique est choisi dans le groupe constitué par un homopolymère ou un copolymère poly(alkylate de vinyle), un homopolymère ou un copolymère poly(alkyléther de vinyle), un homopolymère ou un copolymère poly(acétal de vinyle), et des combinaisons de ceux-ci ; et
l'association d'un principe actif au mélange de polymères miscibles.

38. Procédé de la revendication 37, dans lequel le principe actif est incorporé dans le mélange de polymères miscibles.

39. Procédé de la revendication 37, dans lequel le principe actif est incorporé dans une matrice interne et le mélange de polymères miscibles fournit initialement une barrière à la perméation du principe actif.

40. Procédé de la revendication 37, dans lequel le principe actif est hydrophobe et a un poids moléculaire non supérieur à 1200 g/mol.

# *Fig. 1A*

*Fig. 1B*

# Fig. 1C

# *Fig. 1D*

## Fig. 2

*Fig. 3*

*Fig. 4*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 40347902 P

- US 2004039437 A

**Non-patent literature cited in the description**

- Controlled Drug Delivery: Challenges and Strategies. American Chemical Society, 1997
- **D.W. VAN KREVELEN.** Properties of Polymers. Elsevier

- **D.W.VAN KREVELEN.** Properties of Polymers. Elsevier, 1990
- **M. J. HE ; W. X. CHEN ; X. X. DONG.** *Polymer Physics,* 2000